# EUROPEAN PATENT SPECIFICATION

(11) **EP 1 926 732 B1**
(45) Date of publication and mention of the grant of the patent: **06.11.2013**
(21) Application number: 06820743.0
(22) Date of filing: 01.09.2006
(51) Int. Cl.: C07D 477/20

(54) **A PROCESS FOR THE PREPARATION OF CARBAPENEM COMPOUNDS**
VERFAHREN ZUR HERSTELLUNG VON CARBAPENVERBINDUNGEN
COMPOSES DE CARPAPENEM: OPERATION AMELIOREE DE FABRICATION

(30) Priority: 05.09.2005 IN DE23702005
(43) Date of publication of application: 04.06.2008
(73) Proprietor: Ranbaxy Laboratories Limited, New Delhi 110019 DEL (IN)
(72) Inventor: TEWARI, Neera, Gurgaon, Haryana 122001 (IN); GEORGE, Vinod, Kerala 679333 (IN); MANE, Avinash, Sheshrao, Maharashtra State 413518 (IN); MEERAN, Hashim, Nizar, Poovanathil, Nagoor, Kerala 689645 (IN); PRASAD, Mohan, Gurgaon, Haryana 122001 (IN)
(74) Representative: Cronin, Brian Harold John
(86) International application number: PCT/IB2006/002416
(87) International publication number: WO 2007/029084

(56) References cited:
- EP-A1- 0 528 678
- EP-A1- 1 132 379
- WO-A-98/02439
- WO-A-99/14218
- US-A- 5 527 793
- SUNAGAWA M ET AL: "A NOVEL CARBAPENEM ANTIBIOTIC, SM-7338 STRUCTURE-ACTIVITY RELATIONSHIPS" JOURNAL OF ANTIBIOTICS, JAPAN ANTIBIOTICS RESEARCH ASSOCIATION, TOKYO, JP, vol. 43, no. 5, 1 May 1990 (1990-05-01), pages 519-532, XP002912928 ISSN: 0021-8820
- KYE J S ET AL: "Synthesis and Biological Properties of New 1beta-Methylcarbapenems" BIOORGANIC & MEDICINAL CHEMISTRY LETTERS, OXFORD, GB, vol. 8, no. 13, 7 July 1998 (1998-07-07), pages 1607-1612, XP004137093 ISSN: 0960-894X

## Description

### Field of the Invention

The present invention relates to an improved process for the preparation of carbapenem compounds.

### Background of the Invention

Carbapenem compounds are known for their broad and potent antibacterial activity. A large number of derivatives have been synthesized and investigated for clinical efficacy. Meropenem, ertapenem and doripenem are some of the commercially available carbapenem antibiotics available for treating various bacterial infections.

Thiol side chain compounds of Formulae I and II wherein, 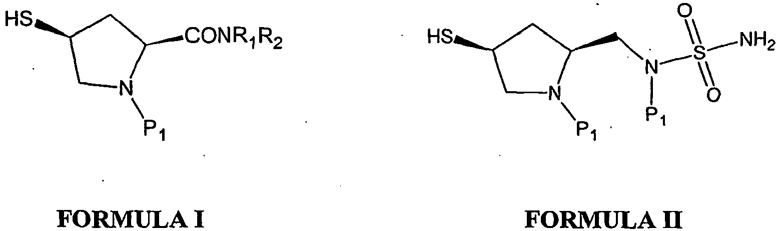 P₁ represents hydrogen or an amino protecting group, and R₁ and R₂ may be hydrogen, C₁₋₅ alkyl, substituted or unsubstituted aryl, or substituted or unsubstituted heteroaryl, are important intermediates in the preparation of carbapenem compounds.

The thiol side chain compounds of the above formulae are further condensed with enolphosphate of Formula III wherein, 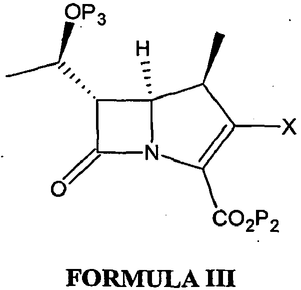 P₂ represents hydrogen or a carboxyl protecting group, P₃ represents hydrogen or hydroxyl protecting group and X represents OP(O)(OR)₂ or OSO₂R, wherein R represents substituted or unsubstituted C₁₋₆ alkyl, aralkyl or aryl, to obtain respective carbapenem antibiotics.

The thiol side chain compounds of Formulae I and II described above may be prepared by S-deprotection of the compounds of Formulae IV and V respectively, 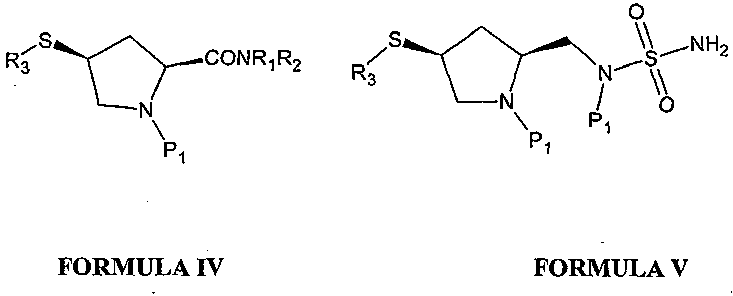 wherein P₁, R₁ and R₂ are as defined above, and R₃ is a thiol protecting group including one or both of acetyl or benzoyl.

U.S. Patent No. 4,943,569 provides a process for the preparation of meropenem, wherein the process involves the reaction between enolphosphate of Formula III, wherein P₂ is p-nitrobenzyl, P₃ is hydrogen and X is diphenoxyphosphoryloxy, and the thiol side chain compound of Formula I, wherein P₁ is p-nitrobenzyloxycarbonyl, and R₁ and R₂ are methyl, followed by deprotection of the protected meropenem intermediate. In this method, the thiol side chain compound of Formula I, wherein P₁ is p-nitrobenzyloxycarbonyl, and R₁ and R₂ are methyl, is prepared by S-deacetylation of the compound of Formula IV, wherein P₁ is p-nitrobenzyloxycarbonyl, R₁ and R₂ are methyl, and R₃ is acetyl, in the presence of aqueous sodium hydroxide and hydrochloric acid.

U.S. Patent No. 5,478,820 provides a similar process for the preparation of ertapenem. The thiol group of the compound of Formula IV, wherein P₁ is p-nitrobenzyloxycarbonyl, R₁ is hydrogen, R₂ is 3-allyloxycarbonylphenyl, and R₃ is acetyl, is deprotected in the presence of 1 M sodium hydroxide.

Yataka Nishino et al., Org. Process Res. Dev. 2003, 7(6), 846-850, provides a process for the preparation of doripenem, where the thiol group of the compound of Formula V, wherein each P₁ independently represents p-nitrobenzyloxycarbonyl or t-butyloxycarbonyl, and R₃ is acetyl, is S-deacetylated in the presence of 98% sulfuric acid.

U.S. Patent No. 5,527,793, PCT Publication No. WO 1999/14218 and Kye J. S. et al, Bioorganic and Medicinal Chemistry Letters, 1998, 8 (13), 1609, provide sodium hydroxide for deprotection of thiol moeities to corresponding deprotected thiol.

EP 0,528,678A1 describes hydrazine hydrate and sodium methoxide for deprotection of acetyl protected thiol compound. PCT Publication No. WO 1998/02439 provide Sulphuric acid for deprotection of thiol moeities to corresponding deprotected thiol. EP 1,132,379A1 provides deprotection of thiol moieties of the pyrrolidin-2-one compounds using acetyl chloride.

Similar processes for the preparation of carbapenem compounds have also been provided in U.S. Patent No. 4,888,344, U.S. Patent No. 5,122,604, Sunagawa M., et al., J. Antibiot (Tokyo), 1990, 43(5), 519-532, Yasuyoshi Iso et al, J. Antibiot (Tokyo), 1996, 49(2), 199-209, and Haruki M., et al., Heterocycles, 1995, 36, 145-159.

The prior art processes involve the preparation of thiol side chain compounds of Formulae I and II by S-deacylation using strong basic or acidic conditions. Such reaction conditions also require a considerable amount of strong base or acid for neutralization of the reaction mixture. Carbapenem ring systems are sensitive to both acidic or basic conditions, and therefore exposure to such conditions generally result in reduced yields and increased impurity levels. However, there remains a need for novel processes that avoid the above problems.

### Summary of the Invention

In one general aspect there is provided a process for the preparation of the compound of Formula VI, 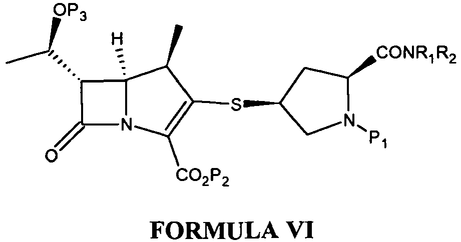 wherein P₁ includes hydrogen or an amino protecting group, P₂ includes hydrogen or a carboxyl protecting group, P₃ includes hydrogen or a hydroxyl protecting group, and R₁ and R₂ include one or more of hydrogen, C₁₋₅ alkyl, substituted or unsubstituted aryl, or substituted or unsubstituted heteroaryl. The process includes the steps of:
a) treating the thiol compound of Formula IV with acetyl chloride, 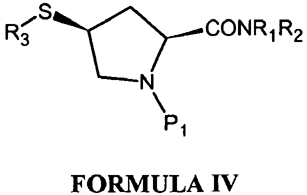 wherein P₁, R₁ and R₂ are as defined above, and R₃ includes a thiol protecting group including acetyl or benzoyl, to form the compound of formula I 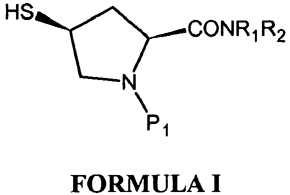 wherein P₁, R₁ and R₂ are as defined above;
b) reacting the compound of Formula I with a compound of Formula III, 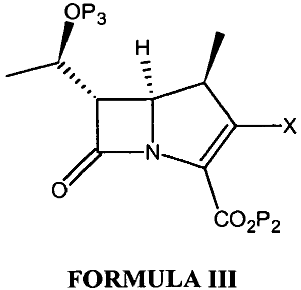 wherein P₂ and P₃ are as defined above and X includes OP(O)(OR)₂ or OSO₂R, R comprises substituted or unsubstituted C₁₋₆ alkyl, aralkyl or aryl, to form the compound of Formula VI; and
c) isolating the compound of Formula VI from the reaction mass thereof.

Embodiments of the process may include one or more of the following features. For example, the compound of Formula VI may be meropenem or ertapenem.

Thiol compound of Formula IV is treated with acetyl chloride. Step a) may be carried out in the presence of one or more organic solvents including C₁₋₅ alkanol, aromatic_hydrocarbon, halogenated hydrocarbon, ketone, ester and ether. Step a) may be carried out under stirring and under reflux temperature.

The compounds of Formula I and Formula II are isolated from the reaction mixture prior to step b). Step b) may further include deprotection of the compounds of Formula VI and Formula VII.

In another general embodiment, there is provided a process for the preparation of the compound of Formula VII, 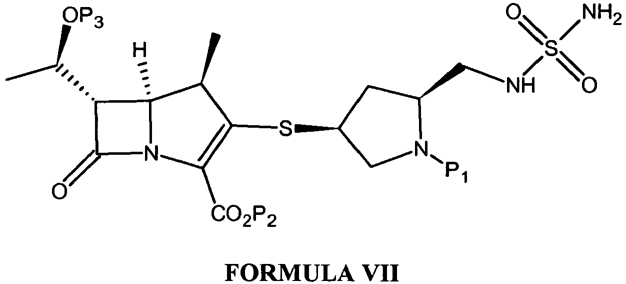 wherein P₁ includes hydrogen or an amino protecting group, P₂ includes hydrogen or a carboxyl protecting group and P₃ includes hydrogen or a hydroxyl protecting group. The process includes the steps of:
a) treating the thiol compound of Formula V with acetyl chloride, 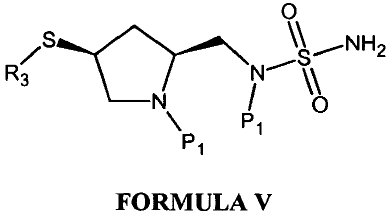 wherein P₁ is as defined above, R₃ includes a thiol protecting group that includes acetyl or benzoyl, to form the compound of formula II, 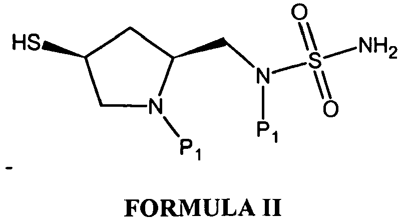 wherein P₁ is as defined above;
b) reacting the compound of Formula II with a compound of Formula III, 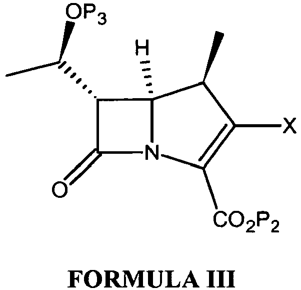 wherein P₂ and P₃ are as defined above and X includes OP(O)(OR)₂ or OSO₂R, R includes substituted or unsubstituted C₁₋₆ alkyl, aralkyl or aryl, to form the compound of Formula VII; and
c) isolating the compound of Formula VII from the reaction mass thereof.

Embodiments of the process may include one or more of the following features. For example, the compound of Formula VII may be doripenem.

The thiol compound of Formula V is treated with acetyl chloride and step a) may be carried out in the presence of one or more organic solvents. The one or more organic solvents may be C₁-₅ alkanol, aromatic hydrocarbon, halogenated hydrocarbon, ketone, ester, ether or mixtures thereof. For example, suitable organic solvents may be a C₁-₅ alkanol.

Step a) may be carried out under stirring and under reflux temperature. The compounds of Formula I and Formula II may be isolated from the reaction mixture prior to step b). Step b) may include the deprotection of the compounds of Formula VI and Formula VII. This deprotection may be carried out in the presence of palladium carbon and an aqueous buffer or the deprotection may be carried out in the presence of non-nucleophilic buffer and in biphasic solvent system.

### Detailed Description of the Invention

It has been found that the S-deprotection of a thiol side chain compound can be carried out in the presence of acetyl chloride. The present method employs only a catalytic quantity of acetyl chloride and does not require the addition of a base or an acid for neutralization. Thus the present process provides final carbapenem compounds with improved purity and good yield.

The term "protecting group," as used herein, refers to protecting groups known in the art and serve the function of blocking carboxyl, amino or hydroxyl groups while the reactions are carried out at other sites of the molecule. Carboxyl protecting groups may include one or more of optionally substituted C₁-C₈ alkyl, optionally substituted C₃-C₈ alkenyl, optionally substituted C₇-C₁₉ aralkyl, optionally substituted C₆-C₁₂ aryl, optionally substituted C₁-C₁₂ amino, optionally substituted C₃-C₁₂ hydrocarbonated silyl, optionally substituted C₃-C₁₂ hydrocarbonated stannyl, and a pharmaceutically active ester forming group. Hydroxyl and amino protecting groups may include one or more of lower alkylsilyl groups, lower alkoxymethyl groups, aralkyl groups, acyl groups, lower alkoxycarbonyl groups, alkenyloxycarbonyl groups and aralkyloxycarbonyl groups.

A first embodiment of the present invention provides a process for the preparation of the compound of Formula VI, 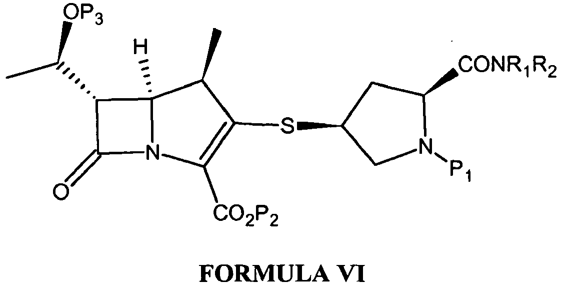 wherein P₁ represents hydrogen or an amino protecting group, P₂ represents hydrogen or a carboxyl protecting group and P₃ represents hydrogen or a hydroxyl protecting group, R₁ and R₂ are hydrogen, C₁₋₅ alkyl, substituted or unsubstituted aryl, or substituted or unsubstituted heteroaryl,
wherein the process comprises:
a) treating the thiol compound of Formula IV with acetyl chloride, 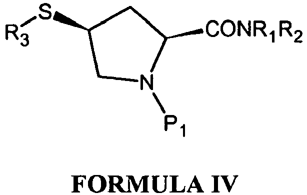 wherein P₁, R₁ and R₂ are as defined above, R₃ is a thiol protecting group one or both of acetyl or benzoyl, to form the compound of formula I 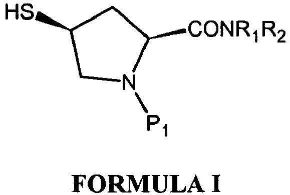 wherein P₁, R₁ and R₂ are as defined above;
b) reacting the compound of Formula I with a compound of Formula III, 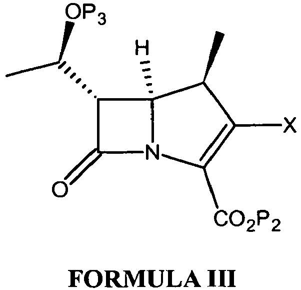 wherein P₂ and P₃ are as defined above and X represents OP(O)(OR)₂ or OSO₂R wherein R represents substituted or unsubstituted C₁₋₆ alkyl, aralkyl or aryl, to form the compound of Formula VI; and
c) isolating the compound of Formula VI from the reaction mass thereof.

Enol-phosphate of Formula III and the thiol side chain of Formula IV may be prepared by processes reported in the abovementioned prior-art. The thiol side chain of Formula IV may be treated with a catalytic quantity of acetyl chloride, in the presence of one or more organic solvents comprising one or more of a C₁₋₅ alkanol, an aromatic hydrocarbon, a halogenated hydrocarbon, a ketone, an ester, an ether or mixtures thereof. The deprotection of the thiol group may be affected by heating the reaction mixture at reflux temperature or by stirring the reaction mixture for sufficient time. The S-deprotected thiol side chain of Formula I may optionally be isolated from the reaction mixture by layer separation and subsequent concentration.

The S-deprotected thiol side chain of Formula I or a reaction mixture comprising the same, and enolphosphate of Formula III may be dissolved in one or more organic solvents and the resultant reaction mixture may be cooled to a temperature less than or equal to about 0 °C. The reaction mixture may be stirred in the presence of one or more organic bases for a sufficient time at the same temperature to effect the coupling reaction. The reaction mixture is subsequently hydrogenated using a palladium catalyst in a biphasic system in presence of a non nucleophilic buffer that includes morpholinopropanesulphonic acid and morpholinoethanesulphonic acid or an aqueous buffer comprising N-methylmorpholine.

After completion of the reaction, the solid product may be isolated from the aqueous layer, washed with one or more organic solvents and dried to obtain the compound of Formula VI.

A second embodiment of the present invention provides a process for the preparation of the compound of Formula VII, 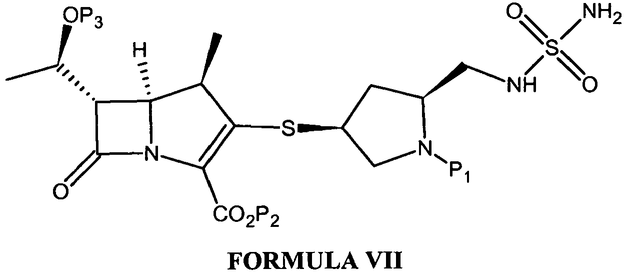 wherein P₁ represents hydrogen or an amino protecting group, P₂ represents hydrogen or a carboxyl protecting group and P₃ represents hydrogen or a hydroxyl protecting group.
The process includes the steps of:
a) treating the thiol compound of Formula V with acetyl chloride, 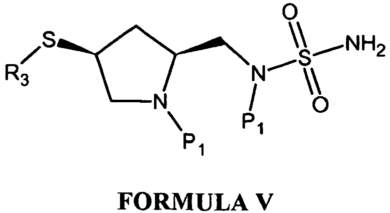 wherein P₁ is as defined above, R₃ is a thiol protecting group comprising one or both of acetyl or benzoyl, to form the compound of Formula II 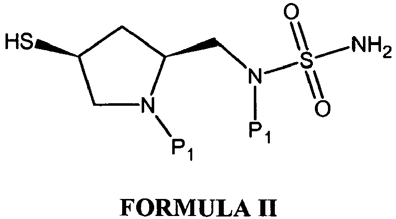 wherein P₁ is as defined above;
b) reacting the compound of Formula II with a compound of Formula III; 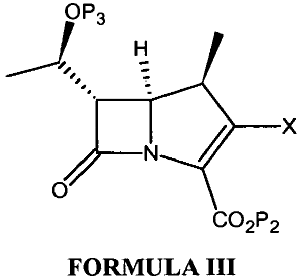 wherein P₂ and P₃ are as defined above, X represents OP(O)(OR)₂ or OSO₂R, and R may be substituted or unsubstituted C₁₋₆ alkyl, aralkyl or aryl, to form the compound of Formula VII; and
c) isolating the compound of Formula VII from the reaction mass thereof.

Enol-phosphate of Formula III and thiol side chain of Formula V may be prepared by processes reported in the abovementioned prior-art. The thiol side chain of Formula V may be treated with a catalytic quantity of acetyl chloride, in the presence of one or more organic solvents. Suitable organic solvents may be one or more of a C₁₋₅ alkanol, an aromatic hydrocarbon, a halogenated hydrocarbon, a ketone, an ester, an ether or mixtures thereof. The thiol group is deprotection by heating the reaction mixture at reflux temperature or by stirring the reaction mixture for sufficient time. The S-deprotected thiol side chain of Formula II may optionally be isolated from the reaction mixture by layer separation and subsequent concentration. The S-deprotected thiol side chain of Formula II or a reaction mixture comprising the same, and enolphosphate of Formula III are dissolved in one or more organic solvents and the resultant reaction mixture is cooled to a temperature less than or equal to about 0°C. The reaction mixture is stirred in the presence of one or more organic bases for a sufficient time at the same temperature to effect the coupling reaction. The reaction mixture is subsequently hydrogenated using a palladium catalyst in a biphasic system in presence of a non nucleophilic buffer. The non nucleophilic buffer may be one or more of morpholinopropanesulphonic acid and morpholinoethanesulphonic acid or an aqueous buffer that includes N-methylmorpholine.

After completion of the reaction, the solid product may be isolated from the aqueous layer, washed with one or more organic solvents and dried to obtain the compound of Formula VII.

### EXAMPLE 1

### PREPARATION OF MEROPENEM:

### a) Preparation of 4-nitrobenzyl (2S,4S)-2-[(dimethylamino)carbonyl]-4-mercaptopyrrolidine-1-carboxylate

4-Nitrobenzyl (2*S*,4*S*)-4-(acetylthio)-2-[(dimethylamino)carbonyl]pyrrolidine-1-carboxylate (50 g) was suspended in methanol (250 mL), followed by the addition of acetyl chloride (10 g). The reaction mixture was stirred for 5 hours at about 25°C and then added to a mixture of methylene chloride (500 mL) and water (500 mL). The organic layer was collected and washed with water (250 mL). The organic layer was concentrated and the residue was recrystallized with isopropylalcohol to obtain the title compound.
Yield: 80 %
PMR (CDCl₃) Data: 8.15 (d, 2H), 7.50 (d, 2H), 5.21 (s, 2H), 4.62 (m, 1H), 4.70 (m, 1H), 4.06 (t, 1H), 3.41 (t, 1H), 3.26 (t, 2H), 3.10 (s, 3H), 2.90 (s, 3H), 2.72 (t, 1H) and 1.88 (1H, br)

### b) Preparation of 4-nitrobenzyl (2S,4S)-2-[(dimethylamino)carbonyl]-4-mercaptopyrrolidine-1-carboxylate

4-Nitrobenzyl (2*S*,4*S*)-4-(acetylthio)-2-[(dimethylamino)carbonyl]pyrrolidine-1-carboxylate (50 g) was suspended in methanol (250 mL), followed by the addition of acetyl chloride (5 g). The reaction mixture was refluxed for 2.5 hours followed by cooling to about 25 °C and then added to a mixture of methylene chloride (500 ml) and water (500 mL). The organic layer was collected and washed with water (250 mL). The organic layer was concentrated and the residue was recrystallized with isopropylalcohol to obtain the title compound.
Yield: 70 %
PMR (CDCl₃) Data: 8.15 (d, 2H), 7.50 (d, 2H), 5.21 (s, 2H), 4.62 (m, 1H), 4.70 (m, 1H), 4.06 (t, 1H), 3.41 (t, 1H), 3.26 (t, 2H), 3.10 (s, 3H), 2.90 (s, 3H), 2.72 (t, 1H) and 1.88 (1H, br)

### c) Preparation of meropenem:

The 4-nitrobenzyl (4*R*,5*R*,6*S*)-3-[(diphenoxyphosphoryl)oxy]-6-[(1*R*)-1-hydroxyethyl]-4-methyl-7-oxo-l-azabicyclo[3.2.0]hept-2-ene-2-carboxylate (50 g) and 4-nitrobenzyl (2*S*,4*S*)-2-[(dimethylamino)carbonyl]-4-mercaptopyrrolidine-1-carboxylate (30 g) obtained from step a) or from step b) were dissolved in a mixture of N,N-dimethylformamide (200 mL) at about 25 °C. The solution was then cooled to -45 °C followed by dropwise addition of diisopropylethylamine (11 g) under stirring while maintaining the temperature between -50 to -45°C. After stirring the reaction mixture for about 1 hour, it was poured into a mixture of ethyl acetate (500 mL) and water (300 mL). The organic layer was separated and added to a mixture of 5 % palladium on carbon (50 g) in aqueous buffer (500 mL) containing N-methylmorpholine and acetic acid (pH about 7.0). The above biphasic reaction mass was hydrogenated for 3 hours under pressure at 20-25 °C. After completion of the reaction, the mixture was filtered and the aqueous layer was separated. The aqueous layer was concentrated by reverse osmosis and tetrahydrofuran was added to the condensate at a temperature of about 5-10 °C. The resultant mixture was stirred for about 5 hours to obtain the title compound as a trihydrate.
Yield: 21.6 g

### EXAMPLE 2

### PREPARATION OF DORIPENEM

### a) Preparation of 4-nitrobenzyl (2S,4S)-2-{[(aminosulfonyl)amino]methyl}-4-mercaptopyrrolidine-1-carboxylate

4-Nitrobenzyl (2S,4S)-4-(acetylthio)-2-{[(aminosulfonyl)(tert-butoxy carbonyl)amino]methyl}pyrrolidine-1-carboxylate (56 g) was suspended in methanol (250 mL), followed by the addition of acetyl chloride (4.13 g). The reaction mixture was refluxed for 2 hours and the completion of the reaction was monitored by thin layer chromatography. After the completion of the reaction, the reaction mixture was cooled to about 25 °C and poured into a mixture of dichloromethane (500 mL) and water (500 mL). The organic layer was collected, washed with water and concentrated to obtain the title compound.
Yield: 50 g

### b) Preparation of doripenem:

N,N-Dimethylformamide (250 mL) was added to the concentrate obtained from step a) followed by the addition of enolphosphate (50 g) at about 25 °C. The resulting solution was cooled to -40 °C and diisopropylethylamine (11 g) was added to this solution drop wise under stirring while maintaining the temperature at between about -40°C to -35 °C. After stirring for 1 hour at the same temperature, the reaction mixture was poured into a mixture of ethylacetate (500 mL) and water (300 mL). The organic layer was separated and added to a mixture of 5 % palladium on carbon (50 g) in an aqueous buffer (500 mL) containing N-methylmorpholine and acetic acid (pH 6.5 to 7.0). The biphasic reaction mass was then hydrogenated for 3 hours under pressure at about 25°C. After the completion of the reaction, the reaction mixture was filtered and the aqueous layer was separated. The analysis of the aqueous layer by HPLC showed the formation of the title compound in an 85 % yield.

## Claims

1. A process for the preparation of the compound of Formula VI, 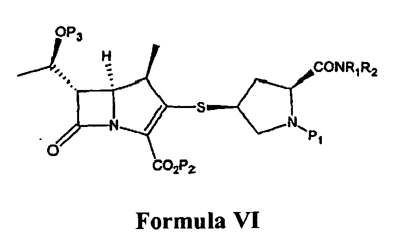 wherein P₁ is an amino protecting group, P₂ is a carboxyl protecting group and P₃ comprises hydrogen or a hydroxyl protecting group, and R₁ and R₂ comprise one or more of hydrogen, C₁₋₅ alkyl, substituted or unsubstituted aryl, or substituted or unsubstituted heteroaryl,
wherein the process comprises:
a) forming a reaction mixture by treating the thiol compound of Formula IV with acetyl chloride, 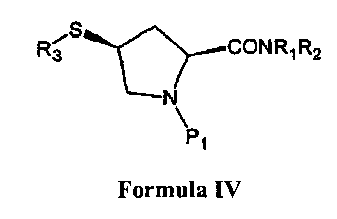 wherein P₁, R₁ and R₂ are as defined above, and R₃ comprises a thiol protecting group comprising of acetyl or benzoyl,
to form the compound of Formula I 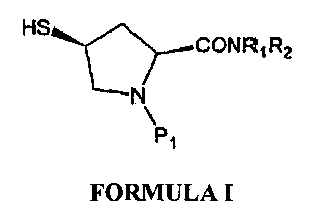 wherein P₁, R₁ and R₂ are as defined above;
b) reacting the compound of Formula I with a compound of Formula III, 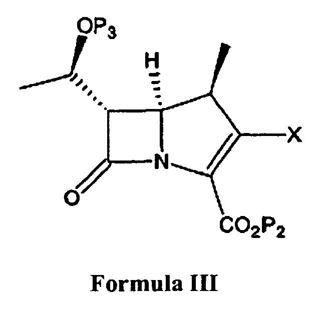 wherein P₂ and P₃ are as defined above and X comprises OP(O)(OR)₂ or OSO₂R, R comprises substituted or unsubstituted C₁₋₆ alkyl, aralkyl or aryl, to form the compound of Formula VI.

2. The process of claim 1, wherein step b) further comprises deprotection of the compounds of Formula VI.

3. The process of claim 2, wherein the compound of Formula VI is deprotected to obtain meropenem.

4. The process of claim 2, wherein the compound of Formula VI is deprotected to obtain ertapenem.

5. The process of claim 1, wherein the compounds of Formula I is isolated from the reaction mixture prior to step b).

6. A process for the preparation of the compound of Formula VII, 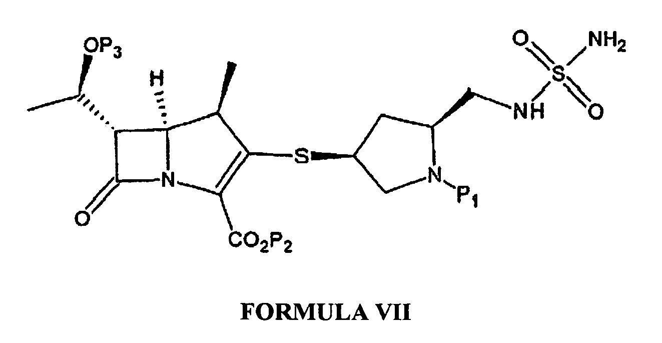 wherein P₁ is an amino protecting group, P₂ is a carboxyl protecting group and P₃ comprises hydrogen or a hydroxyl protecting group, wherein the process comprises:
a) forming a reaction mixture by treating the thiol compound of Formula V with acetyl chloride, 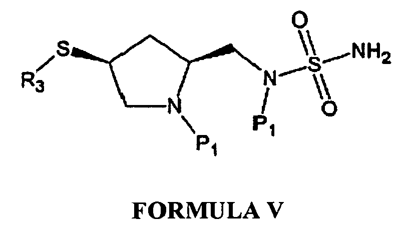 wherein P₁ is as defined above, R₃ comprises a thiol protecting group comprising acetyl or benzoyl, to form the compound of formula II, 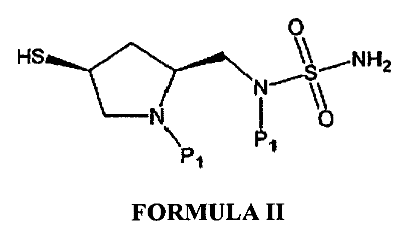 wherein P₁ is as defined above;
b) reacting the compound of Formula II with a compound of Formula III, 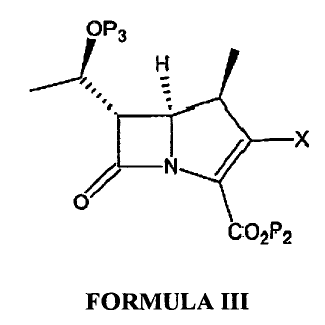 wherein P₂ and P₃ are as defined above and X comprises OP(O)(OR)₂ or OSO2R, R comprises substituted or unsubstituted C₁₋₆ alkyl, aralkyl or aryl, to form the compound of Formula VII.

7. The process of claim6, wherein step b) comprises deprotection of the compounds of Formula VII.

8. The process of claim 7, wherein the compound of Formula VII is deprotected to obtain doripenem.

9. The process of claim6 wherein the compounds of Formula II is isolated from the reaction mixture prior to step b).

## Patentansprüche

1. Verfahren zur Herstellung einer Verbindung der Formel VI, 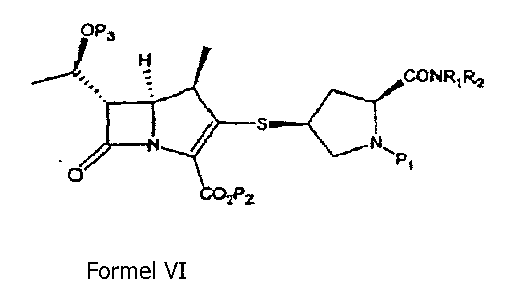 worin P₁ eine Amin schützende Gruppe, P₂ eine Carboxyl schützende Gruppe ist und P₃ Wasserstoff oder eine Hydroxyl schützende Gruppe umfasst, und R₁ und R₂ eines oder mehrere aus Wasserstoff, C₁₋₅-Alkyl,substituiertes oder unsubstituiertes Aryl oder substituiertes oder unsubstituiertes Heteroaryl umfasst,
wobei das Verfahren umfasst:
a) Bilden eines Reaktionsgemisches durch Behandlung der Thiolverbindung der Formel IV mit Acetylchlorid, 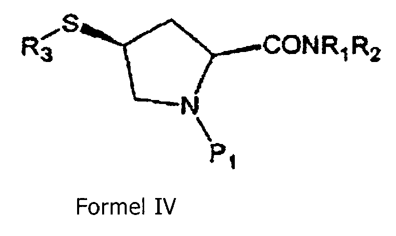 worin P₁, R₁ und R₂ wie oben definiert sind und R₃ eine Thiol schützende Gruppe, umfassend Acetyl oder Benzoyl, umfasst,
unter Bildung einer Verbindung der Formel I, 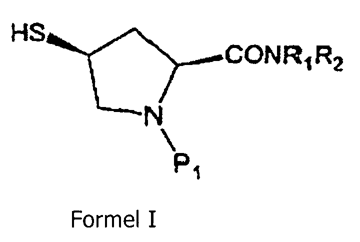 worin P₁, R₁ und R₂ wie oben definiert sind;
b) Umsetzen der Verbindung der Formel I mit einer Verbindung der Formel III, 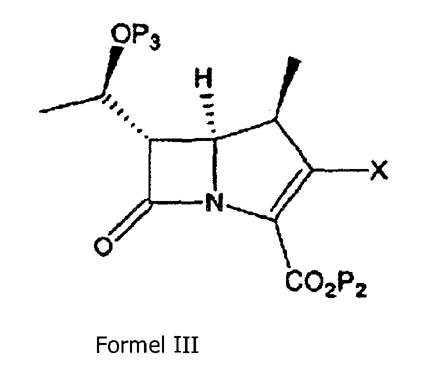 worin P₂ und P₃ wie oben definiert sind und X OP(O)(OR)₂ oder OSO₂R umfasst, R substituiertes oder unsubstituiertes C₁₋₆-Alkyl, Aralkyl oder Aryl umfasst,
unter Bildung einer Verbindung der Formel VI.

2. Verfahren nach Anspruch 1, worin Stufe b) ferner das Entschützen der Verbindungen der Formel VI umfasst.

3. Verfahren nach Anspruch 2, worin die Verbindung der Formel VI zur Gewinnung von Meropenem entschützt wird.

4. Verfahren nach Anspruch 2, worin die Verbindung der Formel VI zur Gewinnung von Ertapenem entschützt wird.

5. Verfahren nach Anspruch 1, worin vor der Stufe b) die Verbindungen der Formel I aus dem Reaktionsgemisch isoliert werden.

6. Verfahren zur Herstellung der Verbindung der Formel VII, 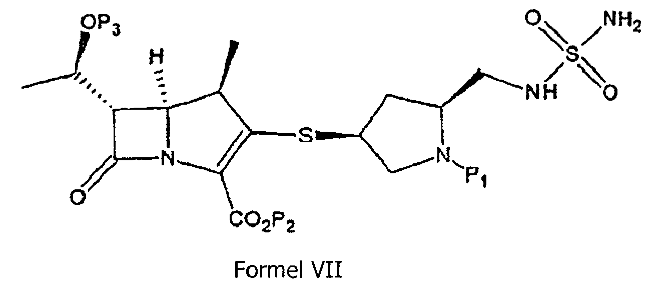 worin P₁ eine Amin schützende Gruppe, P₂ eine Carboxyl schützende Gruppe ist und P₃ Wasserstoff oder eine Hydroxyl schützende Gruppe umfasst, wobei das Verfahren umfasst:
a) Bilden eines Reaktionsgemisch ist durch Behandeln der Thiolverbindung der Formel V mit Acetylchlorid, 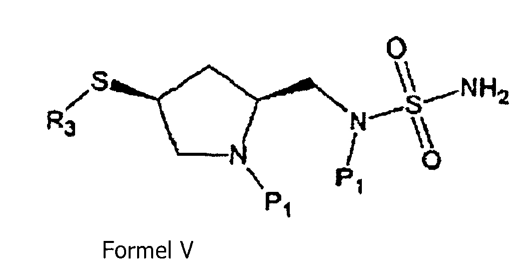 worin P₁ wie oben definiert ist, R₃ eine Thiol schützende Gruppe, umfassend Acetyl oder Benzoyl ist, zur Bildung der Verbindung der Formel II 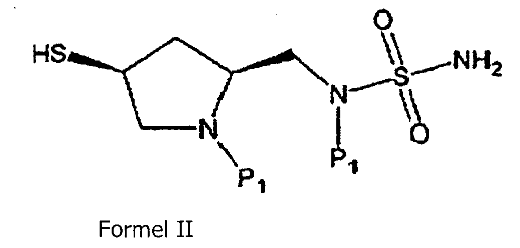 worin P₁ wie oben definiert ist,
b) Umsetzen der Verbindung der Formel II mit einer Verbindung der Formel III, 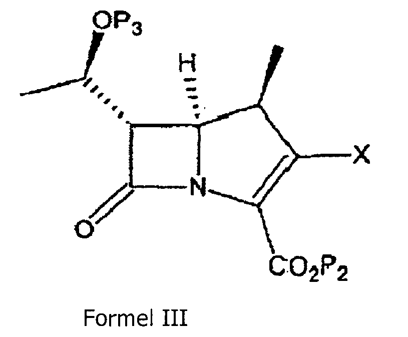 worin P₂ und P₃ wie oben definiert sind und X OP(O)(OR)₂ oder OSO₂R umfasst, R substituiertes oder unsubstituiertes C₁₋₆-Alkyl, Aralkyl oder Aryl umfasst, unter Bildung einer Verbindung der Formel VII.

7. Verfahren nach Anspruch 6, worin die Stufe b) das Entschützen von Verbindungen der Formel VII umfasst.

8. Verfahren nach Anspruch 7, worin die Verbindung der Formel VII zur Gewinnung von Doripenem entschützt wird.

9. Verfahren nach Anspruch 6, worin die Verbindungen der Formel II vor der Stufe b) aus dem Reaktionsgemisch isoliert werden.

## Revendications

1. Procédé pour la préparation du composé de la Formule VI, 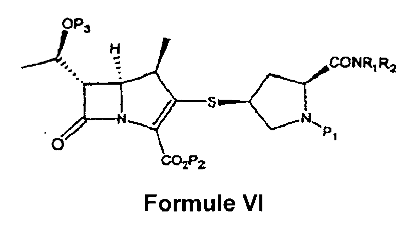 dans laquelle P₁ est un groupe protecteur d'amino, P₂ est un groupe protecteur de carboxyle et P₃ comprend de l'hydrogène ou un groupe protecteur d'hydroxyle, et R₁ et R₂ comprennent un ou plusieurs d'hydrogène, du C₁₋₅ alkyle, de l'aryle substitué ou non substitué, ou de l'hétéroaryle substitué ou non substitué,
dans lequel le procédé comprend :
a) la formation d'un mélange réactionnel par traitement du composé thiol de la Formule IV avec du chlorure d'acétyle, 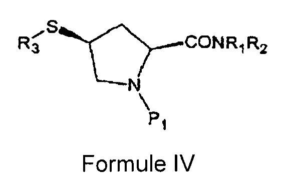 dans laquelle P₁, R₁ et R₂ sont tels que définis précédemment, et R₃ comprend un groupe protecteur de thiol constitué d'acétyle ou de benzoyle pour former le composé de la Formule I 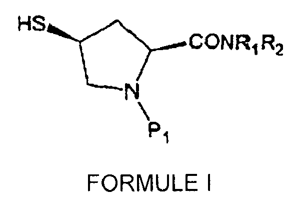 dans laquelle P₁, R₁ et R₂ sont tels que définis précédemment ;
b) la réaction du composé de la Formule I avec un composé de la Formule III, 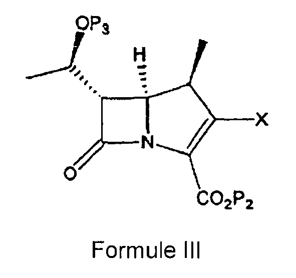 dans laquelle P₂ et P₃ sont tels que définis précédemment et X comprend OP(O)(OR)₂ ou OSO₂R, R comprend un C₁₋₆ alkyle, aralkyle ou aryle substitué ou non substitué pour former le composé de la Formule VI.

2. Procédé selon la revendication 1, dans lequel l'étape b) comprend en outre une déprotection des composés de la Formule VI.

3. Procédé selon la revendication 2, dans lequel le composé de la Formule VI est déprotégé pour obtenir du méropénème.

4. Procédé selon la revendication 2, dans lequel le composé de la Formule VI est déprotégé pour obtenir de l'ertapénème.

5. Procédé selon la revendication 1, dans lequel le composé de la Formule I est isolé du mélange réactionnel avant l'étape b).

6. Procédé pour la préparation du composé de la Formule VII, 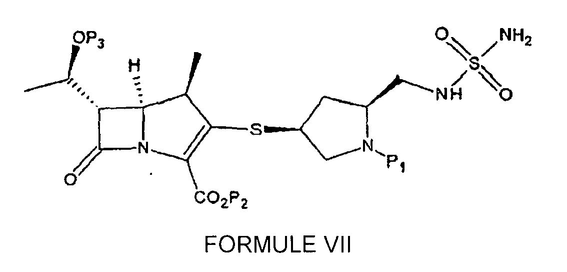 dans laquelle P₁ est un groupe protecteur d'amino, P₂ est un groupe protecteur de carboxyle et P₃ comprend de l'hydrogène ou un groupe protecteur d'hydroxyle, dans lequel le procédé comprend :
a) la formation d'un mélange réactionnel par traitement du composé thiol de la Formule V avec du chlorure d'acétyle, 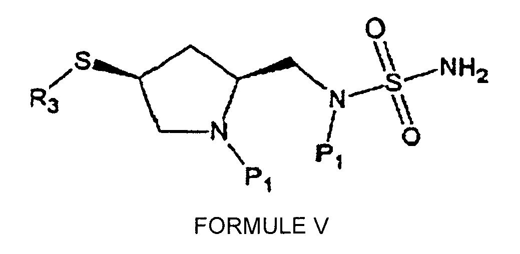 dans laquelle P₁ est tel que défini précédemment, R₃ comprend un groupe protecteur de thiol constitué d'acétyle ou de benzoyle pour former le composé de la Formule II, 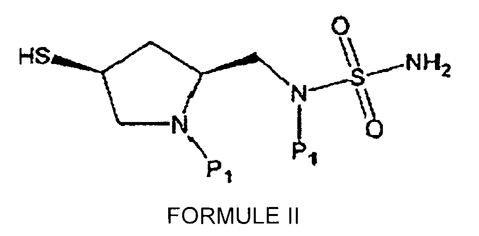 dans laquelle P₁ est tel que défini précédemment ;
b) la réaction du composé de la Formule II avec un composé de la Formule III, 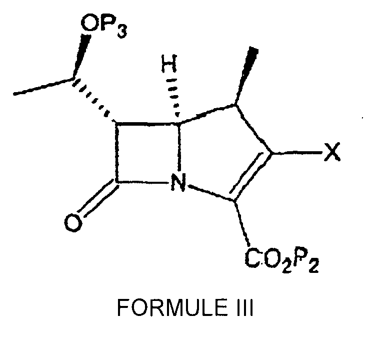 dans laquelle P₂ et P₃ sont tels que définis précédemment et X comprend OP(O)(OR)₂ ou OSO₂R, R comprend un C₁₋₆ alkyle, aralkyle ou aryle substitué ou non substitué pour former le composé de la Formule VII.

7. Procédé selon la revendication 6, dans lequel l'étape b) comprend en outre une déprotection des composés de la Formule VII.

8. Procédé selon la revendication 7, dans lequel le composé de la Formule VII est déprotégé pour obtenir du doripénème.

9. Procédé selon la revendication 6, dans lequel le composé de la Formule II est isolé du mélange réactionnel avant l'étape b).
